# EUROPEAN PATENT APPLICATION

(11) **EP 4 273 881 A2**
(43) Date of publication of application: **08.11.2023**
(21) Application number: 22752992.2
(22) Date of filing: 09.02.2022
(51) Int. Cl.: G16H 50/50, G16H 50/70, G16H 30/40, G16H 30/20, G16H 50/20, G16H 50/30, G16H 10/60, G06N 20/00

(54) **METHOD AND SYSTEM FOR PREDICTING RISK OF OCCURRENCE OF LESION**

(30) Priority: 09.02.2021 KR 20210018405
(71) Applicant: Lunit Inc., Seoul 06241 (KR)
(72) Inventor: KIM, Ki Hwan, Seoul 06241 (KR); NAM, Hyeonseob, Seoul 06241 (KR)
(74) Representative: Studio Torta S.p.A.
(86) International application number: PCT/KR2022/002008
(87) International publication number: WO 2022/173232

(57) **Abstract**

A method performed by one or more processors to predict a risk of occurrence of a lesion is provided, which includes acquiring a medical image of a subject, by using a machine learning model, predicting a possibility of occurrence of a lesion of the subject based on the acquired medical image, and outputting a result of the predicting, in which the machine learning model may be a model trained with a plurality of training medical images and a risk of occurrence of the lesion associated with each training medical image.

## Description

### TECHNICAL FIELD

The present disclosure relates to a method and system for predicting a risk of occurrence of a lesion, and more particularly, to a method and system for providing a patient with information on a risk of occurrence of a lesion based on a medical image of the patient.

### BACKGROUND

Machine learning models can discover hidden characteristics in complex input data and provide meaningful output data. Accordingly, the machine learning models are actively used in various research fields including the medical field. For example, the machine learning models may be used to detect a lesion in a medical image of a patient based on the medical image. In this case, in order to train the machine learning model by supervised learning, a medical image including the lesion and annotation information on the location of the lesion on the medical image may be required as training data. Such training data can be acquired relatively easily by performing annotation work on medical images including lesions.

Meanwhile, the machine learning models are actively used to predict already occurred lesions or diseases from the medical images, but they are not actively used to predict the risk of occurrence of lesions or diseases that are not yet occurred. This is because it is very challenging to find a training method for training a machine learning model to predict the risk of occurrence of a lesion by using medical images of a state in which the disease is not yet occurred. Accordingly, there is a problem that the machine learning model does not provide risk information on diseases that would occur in the future, and is not very helpful in preventing diseases or early detection of diseases through regular screening.

### SUMMARY

In addition, there are provided a method for, a non-transitory computer-readable recording medium storing instructions for, and a device (system) for predicting a risk of occurrence of a lesion.

A method performed by one or more processors to predict a risk of occurrence of a lesion is provided, which may include acquiring a medical image of a subject, by using a machine learning model, predicting a possibility of occurrence of a lesion of the subject based on the acquired medical image, and outputting a result of the predicting, in which the machine learning model is a model trained with a plurality of training medical images and a risk of occurrence of the lesion associated with each of the plurality of training medical images.

The plurality of training medical images may include a high-risk group training medical image and a low-risk group training medical image, and the high-risk group training medical image may include a first training medical image of a lesion region of a patient having the lesion, which was obtained before occurrence of the lesion in the lesion region.

The plurality of training medical images may include a high-risk group training medical image and a low-risk group training medical image, and the high-risk group training medical image may include a second training medical image of a non-lesioned region of a patient having the lesion.

The non-lesioned region of a patient may include at least one of the region opposite to a lesion region or a region surrounding the lesion region.

The high-risk group training medical image may be classified into a plurality of classes according to a degree of risk of occurrence of the lesion.

The machine learning model may include a first classifier trained to classify the plurality of training medical images into a high-risk group training medical image or a low-risk group training medical image, and a second classifier trained to classify the classified high-risk group training medical images into a plurality of classes.

The machine learning model may be a model that is further trained to infer mask annotation information in the training medical images from the training medical images, and the predicting the possibility of occurrence of lesion may include, by using the machine learning model, outputting a region in which the lesion is expected to occur in the acquired medical image.

The medical image may include a plurality of sub medical images, and the predicting the possibility of occurrence of lesion may include extracting a plurality of feature maps output from at least one layer included in the machine learning model by inputting the plurality of sub medical images to the machine learning model, aggregating the plurality of extracted feature maps, and outputting a prediction result on a risk of occurrence of the lesion based on the aggregated plurality of feature maps.

The aggregating the plurality of extracted feature maps may include concatenating or summing the plurality of feature maps.

The outputting the prediction result of the risk of occurrence of the lesion by using the aggregated plurality of feature maps may include outputting the prediction result on the risk of occurrence of the lesion by applying a weight to a specific region within each of the plurality of feature maps.

The medical image may include a mammography image, and the plurality of sub medical images may include two craniocaudal (CC) images and two medial lateral oblique (MLO) images.

The method may further include receiving additional information related to the risk of occurrence of the lesion, and the predicting the possibility of occurrence of the lesion may include, by using the machine learning model, outputting a prediction result on the risk of occurrence of the lesion based on the acquired medical image and the additional information.

The machine learning model may be a model that is further trained to output a reference prediction result on the risk of occurrence of the lesion based on the plurality of training medical images and training additional information.

The method may further include receiving additional information related to a risk of occurrence of the lesion, and the predicting the possibility of occurrence of the lesion may include, by using the machine learning model, outputting a first prediction result on the risk of occurrence of the lesion based on the acquired medical image, by using an additional machine learning model, outputting a second prediction result on the risk of occurrence of the lesion based on the additional information, and generating a final prediction result on the risk of occurrence of the lesion based on the first prediction result and the second prediction result, in which the additional machine learning model is a model trained to output a reference prediction result on the risk of occurrence of the lesion based on training additional information.

The outputting the result of the predicting may further include outputting information related to at least one of medical examination, diagnosis, prevention or treatment based on the prediction result.

There is provided a non-transitory computer-readable recording medium storing instructions for executing the method on a computer.

An information processing system is provided, which may include a memory, and one or more processors connected to the memory and configured to execute one or more computer-readable programs included in the memory, in which the one or more programs may further include instructions for acquiring a medical image of a subject, predicting a possibility of occurrence of a lesion of the subject from the acquired medical image by using a machine learning model, and outputting a result of the predicting, in which the machine learning model is a model trained with a plurality of training medical images and a risk of occurrence of the lesion associated with each of plurality of training medical images.

According to some examples of the present disclosure, the risk of occurrence of a lesion of a patient can be predicted based on the medical images of the patient, and the risk of occurrence of a lesion of the patient can be predicted based on not only the medical images of the patient but also the additional information on the patient, such that the accuracy of prediction can be improved.

According to some examples of the present disclosure, by training the machine learning model using the training medical images obtained from lesion regions of patients with the lesion before the lesion occurred, hidden characteristics of medical images with a high risk of occurrence of the lesion can be learned, and it is thus possible to predict a risk of occurrence of the lesion of a patient.

According to some examples of the present disclosure, by training a machine learning model using the training medical images obtained from at least one of a region opposite to a lesion region, or a region surrounding the lesion region of a patient with the lesion, hidden characteristics of the medical images with a high risk of occurrence of the lesion can be learned, and it is thus possible to predict a risk of occurrence of the lesion of a patient.

According to some examples of the present disclosure, by predicting a risk of occurrence of a lesion of a patient using a plurality of sub medical images obtained from the target region at various locations or angles thereof, it is possible to improve the prediction accuracy.

According to some examples of the present disclosure, since the information on appropriate interventions, schedules, and the like related to treatment, diagnosis, screening or prevention can be provided according to the prediction result on a risk of occurrence of a lesion of a patient and/or the degree of severity, the medical personnel provided with the information can efficiently and effectively manage limited resources (e.g., personnel, equipment, pharmaceuticals, and the like).

According to some examples of the present disclosure, since the information according to the prediction result on the risk of occurrence of a lesion of a patient and/or degree of severity is provided, a high-risk group patient can prevent diseases or detect and treat diseases early through additional screening or short interval screening, and a low-risk group patient can save money and time through long interval screening or the like.

The effects of the present disclosure are not limited to the effects described above, and other effects not described herein can be clearly understood by those of ordinary skill in the art (referred to as "ordinary technician") from the description of the claims.

### BRIEF DESCRIPTION OF THE DRAWINGS

The above and other objects, features and advantages of the present disclosure will be described with reference to the accompanying drawings described below, where similar reference numerals indicate similar elements, but not limited thereto, in which:
FIG. 1 is an exemplary configuration diagram illustrating a system for providing a prediction result on a risk of occurrence of a lesion;
FIG. 2 is a block diagram illustrating an internal configuration of an information processing system;
FIG. 3 is a block diagram of an internal configuration of the user terminal and the information processing system;
FIG. 4 is a diagram illustrating an internal configuration of a processor of the information processing system;
FIG. 5 is a diagram illustrating an example of a training data DB;
FIG. 6 is a diagram illustrating an example of a machine learning model;
FIG. 7 is a diagram illustrating an example of training a machine learning model;
FIG. 8 is a diagram illustrating an example of training a machine learning model;
FIG. 9 is a diagram illustrating an example in which a machine learning model outputs a prediction result on a risk of occurrence of a lesion based on a plurality of sub medical images;
FIG. 10 is a diagram illustrating an example of generating a prediction result on a risk of occurrence of a lesion based on a medical image and additional information;
FIG. 11 is a diagram illustrating an example of generating a prediction result on a risk of occurrence of a lesion based on a medical image and additional information;
FIG. 12 is a diagram illustrating an example of providing medical information based on a prediction result;
FIG. 13 is a diagram illustrating an example of providing a prediction result and medical information based on the prediction result;
FIG. 14 is an exemplary diagram illustrating an artificial neural network model;
FIG. 15 is a flowchart illustrating an example of a method for predicting a risk of occurrence of a lesion; and
FIG. 16 illustrates an exemplary configuration of a system for predicting a risk of occurrence of a lesion.

### DETAILED DESCRIPTION

Hereinafter, example details for the practice of the present disclosure will be described in detail with reference to the accompanying drawings. However, in the following description, detailed descriptions of well-known functions or configurations will be omitted if it may make the subject matter of the present disclosure rather unclear.

In the accompanying drawings, the same or corresponding components are assigned the same reference numerals. In addition, in the following description of various examples, duplicate descriptions of the same or corresponding components may be omitted. However, even if descriptions of elements are omitted, it is not intended that such elements are not included in any example.

Advantages and features of the disclosed examples and methods of accomplishing the same will be apparent by referring to examples described below in connection with the accompanying drawings. However, the present disclosure is not limited to the examples disclosed below, and may be implemented in various different forms, and the examples are merely provided to make the present disclosure complete, and to fully disclose the scope of the invention to those skilled in the art to which the present disclosure pertains.

The terms used herein will be briefly described prior to describing the disclosed example(s) in detail. The terms used herein have been selected as general terms which are widely used at present in consideration of the functions of the present disclosure, and this may be altered according to the intent of an operator skilled in the art, related practice, or introduction of new technology. In addition, in specific cases, certain terms may be arbitrarily selected by the applicant, and the meaning of the terms will be described in detail in a corresponding description of the example(s). Therefore, the terms used in the present disclosure should be defined based on the meaning of the terms and the overall content of the present disclosure rather than a simple name of each of the terms.

As used herein, the singular forms "a," "an," and "the" are intended to include the plural forms as well, unless the context clearly indicates the singular forms. Further, the plural forms are intended to include the singular forms as well, unless the context clearly indicates the plural forms. Further, throughout the description, when a portion is stated as "comprising (including)" a component, it intends to mean that the portion may additionally comprise (or include or have) another component, rather than excluding the same, unless specified to the contrary.

Further, the term "part," "module" or "unit" used herein refers to a software or hardware component, and "part," "module" or "unit" performs certain roles. However, the meaning of the "part," "module" or "unit" is not limited to software or hardware. The "part," "module" or "unit" may be configured to be in an addressable storage medium or configured to play one or more processors. Accordingly, as an example, the "part," "module" or "unit" may include components such as software components, object-oriented software components, class components, and task components, and at least one of processes, functions, attributes, procedures, subroutines, segments of program code, drivers, firmware, micro-codes, circuits, data, database, data structures, tables, arrays, or variables. Furthermore, functions provided in the components and the "parts," "modules" or "units" may be combined into a smaller number of components and "parts," "modules" or "units", or further divided into additional components and "parts," "modules" or "units."

According to an example of the present disclosure, the "part," "module" or "unit" may be implemented as a processor and a memory. The "processor" should be interpreted broadly to encompass a general-purpose processor, a central processing unit (CPU), a Graphic Processing Unit (GPU), a microprocessor, a digital signal processor (DSP), a controller, a microcontroller, a state machine, and so forth. Under some circumstances, the "processor" may refer to an application-specific integrated circuit (ASIC), a programmable logic device (PLD), a field-programmable gate array (FPGA), and so on. The "processor" may refer to a combination for processing devices, e.g., a combination of a DSP and a microprocessor, a combination of a plurality of microprocessors, a combination of one or more microprocessors in conjunction with a DSP core, or any other combination of such configurations. In addition, the "memory" should be interpreted broadly to encompass any electronic component that is capable of storing electronic information. The "memory" may refer to various types of processor-readable media such as random access memory (RAM), read-only memory (ROM), non-volatile random access memory (NVRAM), programmable read-only memory (PROM), erasable programmable read-only memory (EPROM), electrically erasable PROM (EEPROM), flash memory, magnetic or optical data storage, registers, and so on. The memory is said to be in electronic communication with a processor if the processor can read information from and/or write information to the memory. The memory integrated with the processor is in electronic communication with the processor.

In the present disclosure, a "system" may refer to at least one of a server device and a cloud device, but not limited thereto. For example, the system may include one or more server devices. In another example, the system may include one or more cloud devices. In still another example, the system may include both the server device and the cloud device operated in conjunction with each other.

In the present disclosure, a "medical image" may refer to a picture and/or an image captured for diagnosis, treatment, and prevention of a disease, and may refer to a picture and/or an image captured inside/outside the patient's body. Examples of medical image data may include video data and/or image data of all modalities, such as mammography images (MMG), ultrasound images, chest radiograph, computed tomography (CT), positron emission tomography (PET), magnetic resonance imaging (MRI), sonography (Ultrasound, US), functional magnetic resonance imaging (fMRI), digital pathology whole slide image (WSI), digital breast tomosynthesis (DBT). In the present disclosure, a "medical image" may refer to one or more medical images, and in the present disclosure, a "training medical image" may refer to one or more training medical images.

In the present disclosure, "additional information related to the risk of occurrence of a lesion" or "additional information" may include all information that can be acquired from a patient and recorded. For example, the additional information may include lab data and biological data. The additional information is information that medical personnel can obtain from a patient and record the same, and may include information obtained from the patient through taking the medical history of a patient (e.g., address, symptoms, past medical history, family history, smoking status, and the like), physical screening results (e.g., height, blood pressure, heart rate, abdominal examination, and the like of a patient), and additional examination data (e.g., blood test results, electrocardiogram, audiogram, hearing test, and the like). For example, the additional information may include all clinical information of the patient, such as age, weight, family history, height, gender, age at menarche, menopausal status, childbirth history, hormone replacement therapy treatment history, genetic information (e.g., BRCA, BRD, PTEN, TP53, CDH1, SKT11/LKB1, PALB2, and the like), breast density (e.g., density of mammary gland tissue within the breast), blood pressure, body temperature, cough, and underlying disease.

In the present disclosure, the "machine learning model" may include any model that is used to infer an answer to a given input. The machine learning model may include an artificial neural network model including an input layer, a plurality of hidden layers, and an output layer. In an example, each layer may include one or more nodes. For example, the machine learning model may be trained to output a prediction result on a risk of occurrence of a lesion of a patient based on the medical image and/or additional information of the patient. In this case, the label information generated through the annotation task may be used to train the machine learning model. In addition, the machine learning model may include weights associated with a plurality of nodes included in the machine learning model. In an example, the weights may include any parameter associated with the machine learning model. In the present disclosure, the machine learning model may refer to an artificial neural network model, and the artificial neural network model may refer to the machine learning model. The machine learning model herein may be a model trained with various learning methods. For example, various learning methods such as supervised learning, unsupervised learning, reinforcement learning, and the like may be used herein.

In the present disclosure, "learning (training)" may refer to any process of changing weights associated with the machine learning model using the training data and/or the ground-truth labels. The learning (training) may refer to a process of changing or updating weights associated with the machine learning model through one or more of forward propagation and backward propagation of the machine learning model by using the medical images of the learning subject and the ground-truth labels (e.g., a risk of occurrence of a lesion).

In the present disclosure, "annotation" refers to an operation of tagging histological components and the like to a data sample, or refers to the tagged information (that is, annotation) itself. The annotation may be used interchangeably with terms such as tagging, labeling, and so on as used in the art.

In the present disclosure, "each of a plurality of A" may refer to each of all components in the plurality of A, or may refer to each of some of the components in a plurality of A.

In the present disclosure, "similar" may encompass sameness and similarity. For example, when two pieces of information are similar, it may mean that the two pieces of information are the same as or similar to each other.

In the present disclosure, "instructions" may refer to a series of instructions grouped based on functions, which are the components of a computer program and executed by the processor.

In the present disclosure, a "user" may refer to a person who uses a user terminal. For example, the user may include medical personnel, a patient, a researcher, and the like, who are provided with a prediction result on a risk of occurrence of a lesion. In addition, the user may refer to the user terminal, or conversely, the user terminal may refer to the user. That is, the user and the user terminal may be interchangeably used herein.

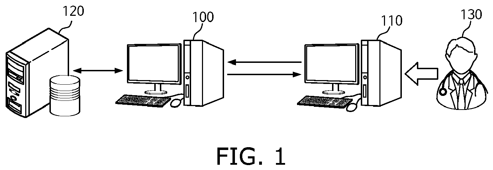

FIG. 1 is an exemplary configuration diagram illustrating a system for providing a prediction result on a risk of occurrence of a lesion of a patient. As illustrated, the system for providing a prediction result on a risk of occurrence of a lesion of a patient may include an information processing system 100, a user terminal 110, and a storage system 120. In an example, the information processing system 100 may be configured to be connected to each of the user terminal 110 and the storage system 120 for communication. While FIG. 1 is a diagram illustrating one user terminal 110, aspects are not limited thereto, and in an exemplary configuration, a plurality of user terminals 110 may be connected to the information processing system 100 for communication. In addition, while the information processing system 100 is illustrated as one computing device in FIG. 1, aspects are not limited thereto, and the information processing system 100 may be configured to process information and/or data in a distributed manner through a plurality of computing devices. In addition, while the storage system 120 is illustrated as a single device in FIG. 1, aspects are not limited thereto, and the system may be configured with a plurality of storage devices or as a system that supports cloud. In addition, respective components of the system for providing a prediction result on a risk of occurrence of the lesion of a patient illustrated in FIG. 1 represent functional components that can be divided on the basis of functions, and in an actual physical environment, a plurality of components may be implemented as being incorporated into each other.

The information processing system 100 and the user terminal 110 are any computing devices used to generate and they provide a prediction result on a risk of occurrence of a lesion of a patient. In an example, the computing device may refer to any type of device equipped with a computing function, and may be a notebook, a desktop, a laptop, a server, a cloud system, and the like, for example, but is not limited thereto.

The information processing system 100 may receive a medical image of the patient and/or additional information of the patient. In this case, the additional information of the patient may include clinical data, lab data, and/or biological data of the patient. For example, the information processing system 100 may receive the medical image of the patient and/or the additional information of the patient from the storage system 120 (e.g., hospital system, electronic medical records, prescription delivery system, medical imaging system, examination information system, other local/cloud storage system, and the like) and/or the user terminal 110. The information processing system 100 may generate a prediction result on a risk of occurrence of the lesion of a patient and provide the generated prediction result to a user 130 through the user terminal 110.

The information processing system 100 may use a machine learning model to generate and output a prediction result on a risk of occurrence of a lesion of a patient based on the medical image of the patient and/or the additional information of the patient. In this case, the prediction result on a risk of occurrence of the lesion of a patient may include information in which the risk of occurrence of the lesion is expressed by a means (such as a numerical value or color) that can express the degree of severity of the risk, information that is classified into a plurality of classes (high risk, intermediate risk, low risk) according to the degree of risk of occurrence of the lesion, etc.

Additionally or alternatively, the information processing system 100 may provide information related to at least one of medical examination, diagnosis, prevention, or treatment, based on the prediction result on the risk of occurrence of the lesion. For example, the information processing system 100 may provide information on a prognosis of a patient, necessary intervention (e.g., treatment, diagnosis, test, prevention policy and timing) required of the patient in a specific situation, drug response, or the like, based on the prediction result on the risk of occurrence of the lesion. As a specific example, the information processing system 100 may provide a personalized screening schedule according to the degree of risk of occurrence of the lesion. The information processing system 100 may recommend additional examinations (e.g., MRI, CT scans, or the like) to a patient with a high risk of occurrence of the lesion, and may provide a screening schedule for routine screening at short intervals. On the other hand, a patient with a low risk of occurrence of the lesion may be provided with a screening schedule for routine screening at long intervals.

The information processing system 100 may provide a prediction result on a risk of occurrence of a lesion of a patient and/or various medical information generated based on the prediction result to the user terminal 110. The user terminal 110 may receive the prediction result on the risk of occurrence of the lesion of the patient and/or various medical information generated based on the prediction result from the information processing system 100, and output the received information through a display device. That is, the user (e.g., medical personnel, patient, researcher, etc.) 130 may perform medical interventions and/or clinical decisions on a patient based on the prediction result on a risk of occurrence of the lesion of a patient and/or various medical information generated based on the prediction result.

The storage system 120 is a device or a cloud system that stores and manages the medical images and the additional information associated with patients and various data associated with the machine learning model so as to provide a prediction result on a risk of occurrence of a lesion of a patient. For efficient data management, the storage system 120 may store and manage various types of data using a database. In this example, the various data may include any data associated with the machine learning model, and include, for example, a file/meta information of the training data, a file/meta information of the target data, label information for the target data as a result of the annotation work, data related to the annotation work, a machine learning model (e.g., an artificial neural network model), and the like, but are not limited thereto. While FIG. 1 shows the information processing system 100 and the storage system 120 as separate systems, the present disclosure is not limited thereto, and they may be incorporated into one system.

According to some examples of the present disclosure, the user 130 may be provided with a prediction result on a risk of occurrence of a lesion of a patient and/or various medical information based on the prediction result. The user 130 may be medical personnel or a patient himself/herself. For example, when the user 130 is medical personnel, the medical personnel can take necessary intervention for the patient and, with the help of the various medical information provided, can make accurate clinical decision on the patient.

In addition, according to some examples, since the information on appropriate interventions, schedules, etc. related to treatment, diagnosis, screening or prevention can be provided according to prediction results on a risk of occurrence of a lesion of a patient and/or the degree of severity, the medical personnel provided with the information can efficiently and effectively manage limited resources (e.g., personnel, equipment, pharmaceuticals, and the like), and on the patient's side, a high-risk group patient provided with the information can prevent disease or detect disease early through additional screening or short interval screening, while a low-risk group patient provided with the information can save money and time through long interval screening, etc.

In the following description, mammography images are described as a specific example of medical images, and the risk of occurrence of breast cancer is described as a specific example of the risk of occurrence of a lesion, but this is only for a clear understanding of the present disclosure, and the scope of the present disclosure is not limited thereto. That is, according to the present disclosure, the risk of occurrence of any lesion may be predicted based on any medical image.

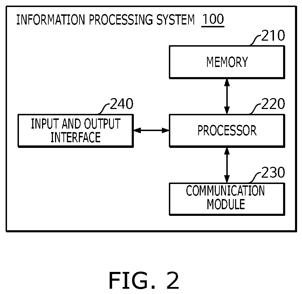

FIG. 2 is a block diagram illustrating an internal configuration of the information processing system 100. The information processing system 100 may include a memory 210, a processor 220, a communication module 230, and an input and output interface 240. As illustrated in FIG. 2, the information processing system 100 may be configured to communicate information and/or data through a network by using the communication module 230. The information processing system 100 may be formed of at least one device including the memory 210, the processor 220, the communication module 230, and the input and output interface 240.

The memory 210 may include any non-transitory computer-readable recording medium. The memory 210 may include a permanent mass storage device such as random access memory (RAM), read only memory (ROM), disk drive, solid state drive (SSD), flash memory, and so on. In another example, a non-destructive mass storage device such as ROM, SSD, flash memory, disk drive, and so on may be included in the information processing system 100 as a separate permanent storage device that is distinct from the memory 210. In addition, the memory 210 may store an operating system and at least one program code (e.g., a code for predicting a risk of occurrence of a lesion that is installed and driven in the information processing system 100).

These software components may be loaded from a computer-readable recording medium separate from the memory 210. Such a separate computer-readable recording medium may include a recording medium directly connectable to the information processing system 100, and may include a computer-readable recording medium such as a floppy drive, a disk, a tape, a DVD/CD-ROM drive, a memory card, and the like, for example. In another example, the software components may be loaded into the memory 210 through the communication module 230 rather than the computer-readable recording medium. For example, the at least one program may be loaded into the memory 210 based on a computer program (e.g., a program or the like for predicting a risk of occurrence of a lesion, etc.) installed by the files provided by the developers, or by a file distribution system that distributes an installation file of an application through the communication module 230.

The processor 220 may be configured to process the commands of the computer program by performing basic arithmetic, logic, and input and output computations. The commands may be provided to a user terminal (not illustrated) or another external system by the memory 210 or the communication module 230. For example, the processor 220 may receive a medical image, and, using a machine learning model, generate a prediction result on a risk of occurrence of a lesion based on the received medical image and provide the result.

The communication module 230 may provide a configuration or function for the user terminal (not illustrated) and the information processing system 100 to communicate with each other through a network, and may provide a configuration or function for the information processing system 100 to communicate with an external system (e.g., a separate cloud system). For example, control signals, commands, data, and the like provided under the control of the processor 220 of the information processing system 100 may be transmitted to the user terminal and/or the external system through the communication module 230 and the network through the communication module of the user terminal and/or an external system. For example, the prediction result generated by the information processing system 100 and/or the medical information generated based on the prediction result may be transmitted to the user terminal and/or the external system through the communication module of the user terminal and/or the external system via the communication module 230 and the network. In addition, the user terminal and/or the external system receiving the prediction result and/or the medical information generated based on the prediction result may output the received information through a device capable of outputting a display.

In addition, the input and output interface 240 of the information processing system 100 may be a means for interfacing with a device (not illustrated) for inputting or outputting, which may be connected to the information processing system 100 or included in the information processing system 100. In FIG. 2, the input and output interface 240 is illustrated as a component configured separately from the processor 220, but aspects are not limited thereto, and the input and output interface 240 may be configured to be included in the processor 220. The information processing system 100 may include more components than those illustrated in FIG. 2. Meanwhile, most of the related components may not necessarily require exact illustration.

The processor 220 of the information processing system 100 may be configured to manage, process, and/or store the information and/or data received from a plurality of user terminals and/or a plurality of external systems. The processor 220 may receive the medical image from the user terminal and/or the external system. The processor 220 may use a machine learning model to generate a prediction result on a risk of occurrence of a lesion based on the received medical images and/or various medical information based on the prediction result, and output the generated information through a device capable of outputting a display, which is connected to the information processing system 100.

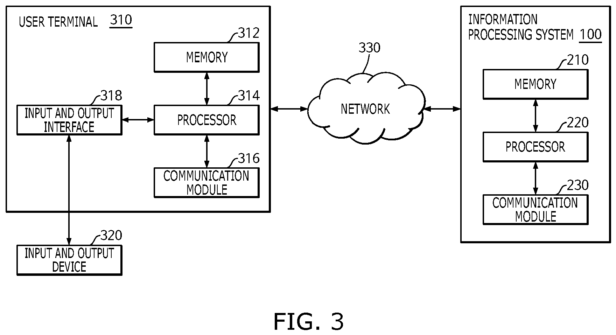

FIG. 3 is a block diagram of an internal configuration of a user terminal 310 and the information processing system 100. In the following description of FIG. 3, description overlapping with the above description with reference to FIG. 2 will be briefly summarized or omitted, and the description will focus on the additional configuration in FIG. 3. The user terminal 310 may refer to any computing device that is capable of executing an application or a web browser that provides a service of predicting a risk of occurrence of a lesion, and the like and capable of wired/wireless communication, and may include a mobile phone terminal, a tablet terminal, a PC terminal, and the like, for example. As illustrated, the user terminal 310 may include a memory 312, a processor 314, a communication module 316, and an input and output interface 318. As illustrated in FIG. 3, the user terminal 310 and the information processing system 100 may be configured to communicate information and/or data through the network 330 using respective communication modules 316 and 336. In addition, an input and output device 320 may be configured to input information and/or data to the user terminal 310 or output information and/or data generated from the user terminal 310 through the input and output interface 318.

The memories 312 and 210 may include any non-transitory computer-readable recording medium. The memories 312 and 210 may include a permanent mass storage device such as random access memory (RAM), read only memory (ROM), disk drive, solid state drive (SSD), flash memory, and so on. As another example, a non-destructive mass storage device such as ROM, SSD, flash memory, disk drive, and so on may be included in the user terminal 310 or the information processing system 100 as a separate permanent storage device that is distinct from the memory. In addition, an operating system and at least one program code (e.g., a code installed and driven in the user terminal 310 for predicting a risk of occurrence of a lesion, etc.) may be stored in the memories 312 and 210.

These software components may be loaded from a computer-readable recording medium separate from the memories 312 and 210. Such a separate computer-readable recording medium may include a recording medium directly connectable to the user terminal 310 and the information processing system 100, and may include a computer-readable recording medium such as a floppy drive, a disk, a tape, a DVD/CD-ROM drive, a memory card, and so on, for example. As another example, the software components may be loaded into the memories 312 and 210 through the communication modules rather than the computer-readable recording medium. For example, at least one program may be loaded into the memories 312 and 210 based on a computer program installed by files provided by developers or a file distribution system that distributes an installation file of an application via the network 330.

The processors 314 and 220 may be configured to process the instructions of the computer program by performing basic arithmetic, logic, and input and output operations. The instructions may be provided to the processors 314 and 220 from the memories 312 and 210 or the communication modules 316 and 230. For example, the processors 314 and 220 may be configured to execute the received instructions according to a program code stored in a recording device such as the memories 312 and 210.

The communication modules 316 and 230 may provide a configuration or function for the user terminal 310 and the information processing system 100 to communicate with each other through the network 330, and may provide a configuration or function for the user terminal 310 and/or the information processing system 100 to communicate with another user terminal or another system (e.g., a separate cloud system or the like). For example, a request or data (e.g., data associated with the request to predict a risk of occurrence of a lesion, etc.) generated by the processor 314 of the user terminal 310 according to the program code stored in the recording device such as the memory 312 and the like may be sent to the information processing system 100 through the network 330 under the control of the communication module 316. Conversely, a control signal or a command provided under the control of the processor 220 of the information processing system 100 may be received by the user terminal 310 through the communication module 316 of the user terminal 310 through the communication module 230 and the network 330. For example, the user terminal 310 may receive data and the like associated with the prediction result on a risk of occurrence of a lesion from the information processing system 100.

The input and output interface 318 may be a means for interfacing with the input and output device 320. As an example, the input device may include a device such as a camera including an audio sensor and/or an image sensor, a keyboard, a microphone, a mouse, and so on, and the output device may include a device such as a display, a speaker, a haptic feedback device, and so on. As another example, the input and output interface 318 may be a means for interfacing with a device such as a touch screen or the like that integrates a configuration or function for performing inputting and outputting. For example, when the processor 314 of the user terminal 310 processes the instructions of the computer program loaded into the memory 312, a service screen or the like, which is configured with the information and/or data provided by the information processing system 100 or another user terminals, may be displayed on the display via the input and output interface 318. While FIG. 3 illustrates that the input and output device 320 is not included in the user terminal 310, aspects are not limited thereto, and an input and output device may be configured as one device with the user terminal 310. In FIG. 3, the input and output interface 318 is illustrated as a component configured separately from the processor 314, but aspects are not limited thereto, and the input and output interface 318 may be configured to be included in the processor 314. In another example, the information processing system 100 may also be configured to include an input/output interface (not illustrated). In this case, the input and output interface of the information processing system 100 may be a means for interfacing with a device (not illustrated) for inputting or outputting which may be connected to, or included in the information processing system 100.

The user terminal 310 and the information processing system 100 may include more than those components illustrated in FIG. 3. Meanwhile, most of the related components may not necessarily require exact illustration. The user terminal 310 may be implemented to include at least a part of the input and output device 320 described above. In addition, the user terminal 310 may further include other components such as a transceiver, a Global Positioning System (GPS) module, a camera, various sensors, a database, and the like. For example, if the user terminal 310 is a smartphone, it may include components generally included in the smartphone. For example, it may be implemented such that various components such as an acceleration sensor, a gyro sensor, an image sensor, a proximity sensor, a touch sensor, an illuminance sensor, a camera module, various physical buttons, buttons using a touch panel, input and output ports, a vibrator for vibration, and so on may be further included in the user terminal 310. The processor 314 of the user terminal 310 may be configured to operate an application or the like that provides a service for predicting a risk of occurrence of a lesion. A code associated with the application and/or program may be loaded into the memory 312 of the user terminal 310.

While the program for the application or the like that provides a service for predicting a risk of occurrence of a lesion is being operated, the processor 314 may receive text, image, video, audio, and/or action, and so on inputted or selected through the input device such as a touch screen connected to the input and output interface 318, a keyboard, a camera including an audio sensor and/or an image sensor, a microphone, and so on, and store the received text, image, video, audio, and/or action, and so on in the memory 312, or provide the same to the information processing system 100 through the communication module 316 and the network 330. For example, the processor 314 may receive a user input requesting a prediction on a risk of occurrence of a lesion on a medical image, and provide the result to the information processing system 100 through the communication module 316 and the network 330.

The processor 314 of the user terminal 310 may be configured to manage, process, and/or store the information and/or data received from the input and output device 320, another user terminal, the information processing system 100 and/or a plurality of external systems. The information and/or data processed by the processor 314 may be provided to the information processing system 100 via the communication module 316 and the network 330. The processor 314 of the user terminal 310 may transmit the information and/or data to the input and output device 320 via the input and output interface 318 to output the same. For example, the processor 314 may display the received information and/or data on a screen of the user terminal.

The processor 220 of the information processing system 100 may be configured to manage, process, and/or store information and/or data received from a plurality of user terminals 310 and/or a plurality of external systems. The information and/or data processed by the processor 220 may be provided to the user terminals 310 via the communication module 230 and the network 330.

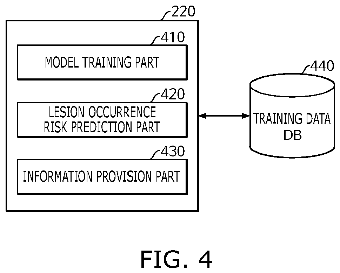

FIG. 4 is a diagram illustrating an internal configuration of the processor 220 of the information processing system. The processor 220 may include a model training part 410, a lesion occurrence risk prediction part 420, and an information provision part 430. Although the internal components of the processor 220 have been described separately for each function in FIG. 4, this does not necessarily mean that they are physically separated. In addition, the internal configuration of the processor 220 illustrated in FIG. 3 is only an example, and it is not intended to depict essential configurations only. Accordingly, in some examples, the processor 220 may be implemented differently, such as by additionally including components other than those internal components illustrated, or by omitting some of the illustrated components.

The processor 220 may acquire a medical image of a patient who is a subject for the prediction on a risk of occurrence of a lesion. In this example, the medical image is a picture and/or image captured for diagnosis, treatment, and prevention of a disease, and may refer to a picture and/or image captured inside/outside the patient's body. The medical image may include a plurality of sub medical images. For example, the medical image may include a mammography image, and the plurality of sub medical images may include two craniocaudal (CC) images and two medial lateral oblique (MLO) images.

Additionally, the processor 220 may further receive additional information related to the risk of occurrence of the lesion. In this case, the additional information may include clinical data, lab data and/or biological data. As a specific example, for the prediction on a risk of occurrence of breast cancer, the additional information may include at least one of the age, weight, family history, height, gender, age at menarche, menopausal status, birth history, hormone replacement therapy treatment history, genetic information of a patient (e.g., BRCA, BRD, PTEN, TP53, CDH1, SKT11/LKB 1, PALB2, and the like), and breast density.

The images and/or information and the like may be received from a storage system connected to or in communicable with an information processing system (e.g., hospital system, electronic medical records, prescription delivery system, medical imaging system, examination information system, other local/cloud storage system, and the like), an internal memory, and/or a user terminal. The received medical image and/or additional information may be provided to the lesion occurrence risk prediction part 420 and used for generating a prediction result on a risk of occurrence of a lesion.

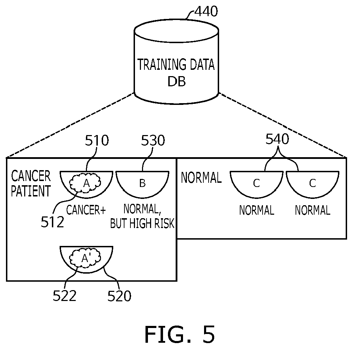

The model training part 410 may receive training data necessary for training the model and train the machine learning model. The training data necessary for training the model may be stored in a training data DB 440. The training data DB 440 may include a high-risk group training medical image, a low-risk group training medical image, training additional information, a reference prediction result on a risk of occurrence of a lesion associated with each training medical image and/or each training additional information, mask annotation information for the high-risk group training medical image, and the like. Examples of the training data stored in the training data DB 440 will be described below in detail with reference to FIG. 5.

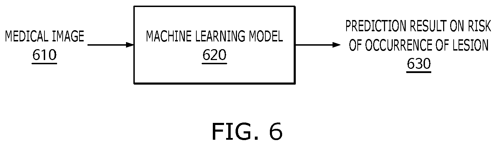

The model training part 410 may train the machine learning model to output a reference prediction result on a risk of occurrence of a lesion from each of a plurality of training medical images including the high-risk group training medical image and the low-risk group training medical image. Additionally, the model training part 410 may further train the machine learning model such that the machine learning model infers the mask annotation information in the high-risk group training medical image from the high-risk group training medical image. A specific example in which the model training part 410 trains the machine learning model to output the reference prediction result on a risk of occurrence of the lesion from each of a plurality of training medical images will be described below in detail with reference to FIG. 6.

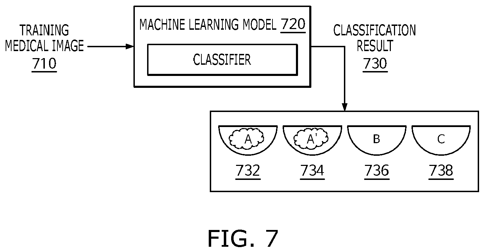

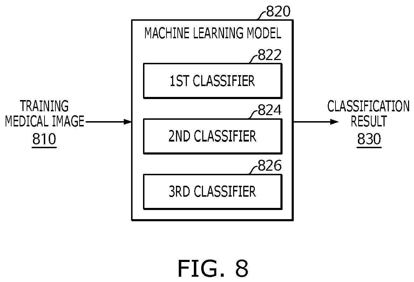

The training medical images may be classified into a plurality of classes according to the degree of risk of occurrence of the lesion. In this case, the model training part 410 may train the machine learning model to classify a plurality of training medical images into a plurality of classes. A specific example in which the model training part 410 trains the machine learning model to classify a plurality of training medical images into a plurality of classes will be described below in detail with reference to FIGS. 7 and 8.

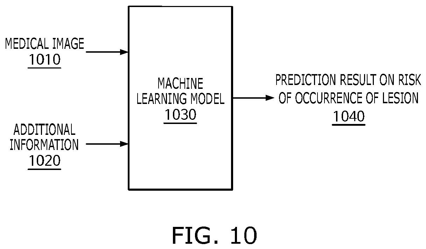

Additionally or alternatively, the model training part 410 may train the machine learning model to output the reference prediction result on a risk of occurrence of the lesion by using a plurality of training medical images and the training additional information. An example in which the model training part 410 trains the machine learning model to output the reference prediction result on the risk of occurrence of the lesion by using each training medical image and the training additional information will be described below in detail with reference to FIGS. 10 to 11.

The lesion occurrence risk prediction part 420 may use the trained machine learning model to generate or output the prediction result on a risk of occurrence of the lesion. The machine learning model may be a model trained by the model training part 410. For example, the lesion occurrence risk prediction part 420 may use the machine learning model to generate a prediction result on a risk of occurrence of the lesion based on the medical image. Additionally, the lesion occurrence risk prediction part 420 may use the machine learning model to generate information on a region (e.g., one or more pixel regions) in which the lesion is expected to occur in the received medical image. An example in which the lesion occurrence risk prediction part 420 uses the machine learning model to generate a prediction result on a risk of occurrence of the lesion based on the medical image will be described below in detail with reference to FIG. 6.

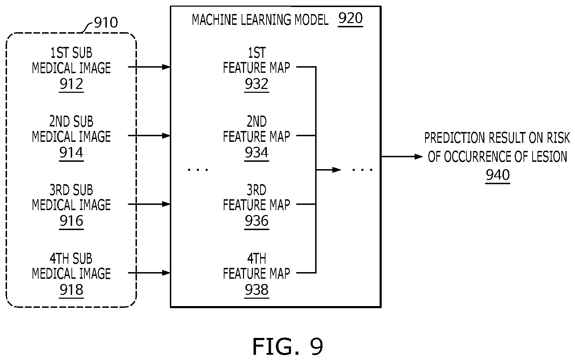

The medical image may include a plurality of sub medical images. In this case, the lesion occurrence risk prediction part 420 may input a plurality of sub medical images to the machine learning model and extract a plurality of feature maps output from at least one layer included in the machine learning model, and aggregate a plurality of extracted feature maps and use a plurality of aggregated feature maps to generate a prediction result on a risk of occurrence of the lesion. An example in which the lesion occurrence risk prediction part 420 generates a prediction result on a risk of occurrence of the lesion based on a plurality of sub medical images will be described below in detail with reference to FIG. 9.

Additionally or alternatively, the lesion occurrence risk prediction part 420 may use the received medical image and additional information to generate a prediction result on a risk of occurrence of the lesion. For example, the lesion occurrence risk prediction part 420 may use one machine learning model to generate a prediction result on a risk of occurrence of the lesion based on the received medical image and additional information, or use a plurality of models to generate a prediction result on a risk of occurrence of the lesion based on the received medical images and additional information. An example in which the lesion occurrence risk prediction part 420 uses the received medical image and additional information to generate a prediction result on a risk of occurrence of the lesion will be described below in detail with reference to FIGS. 10 to 11.

Additionally, the lesion occurrence risk prediction part 420 may be configured to output information associated with the generated prediction result through an output device connected to the information processing system or through an output device of the user terminal.

The information provision part 430 may provide information related to at least one of medical examination, diagnosis, prevention, or treatment based on the prediction result generated by the lesion occurrence risk prediction part 420. For example, for the information related to at least one of medical examination, diagnosis, prevention, or treatment, the information provision part 430 may provide, based on the prediction result, prognosis of a patient, necessary intervention (e.g., treatment, diagnosis, examination, prevention policy and timing) required of a patient in a specific situation, or information on drug response and the like. As a specific example, the information provision part 430 may provide a personalized suitable screening schedule according to the degree of risk of occurrence of the lesion. The information provision part 430 may recommend additional examinations (e.g., MRI, CT scans, or the like) to a patient with a high risk of occurrence of the lesion, and may provide a screening schedule for routine screening at short intervals. On the other hand, the information provision part 430 may provide a screening schedule for routine screening at long intervals to a patient with a low risk of occurrence of the lesion.

The information provision part 430 may provide information related to at least one of medical examination, diagnosis, prevention, or treatment to the user terminal, and the provided information may be output through a screen of the user terminal.

At least some of the processes described above as being performed by the processor 220 of the information processing system may be performed by the processor of the user terminal. For example, at least some of the prediction result and/or medical information generated by the processor 220 of the information processing system may be generated by the user terminal.

FIG. 5 is a diagram illustrating an example of the training data DB 440. The training data DB 440 may include training data for training a machine learning model. The training data DB 440 may be included in the information processing system 100 or communicatively connected to the information processing system 100.

The training data may include a reference prediction result of each of high-risk group training medical images, a low-risk group training medical images, and training medical images. The high-risk group training medical image may refer to a medical image of a reference patient having a relatively high risk of occurrence of a target disease, and the low-risk group training medical image may refer to a medical image of a reference patient having a relatively low risk of occurrence of a target disease. The reference prediction result of each training medical image may include the degree of risk of occurrence of the lesion for each training medical image. For example, the reference prediction result may include information in which the risk of occurrence of the lesion is expressed by a means (e.g., numerical value, color, or the like) that can express the degree of severity of the risk, information that is classified into a plurality of classes (e.g., high risk, intermediate risk, low risk) according to the degree of risk of occurrence of the lesion, etc. The reference prediction result of each training medical image may be included as annotation information labeled in each training medical image.

The high-risk group training medical image and/or the low-risk group training medical image may be classified into a plurality of classes according to the degree of risk of occurrence of the lesion. For example, the high-risk group training medical image may include at least one of a training medical image 510 of the lesion region of a patient having the lesion, a training medical image 520 of the lesion region of a patient having the lesion, which was obtained before occurrence of the lesion in the lesion region, or a training medical image 530 of a non-lesioned region of a patient having the lesion. In this example, the training medical image 530 of a non-lesioned region of a patient having the lesion may include a training medical image 530 of at least one of a region opposite to, or a region surrounding the lesion area in a patient having the lesion. Since the non-lesioned region of a patient having the lesion tends to have a higher possibility of occurrence of the lesion than the same region of a person not having the lesion, the training medical image 530 of a non-lesioned region of the patient having the lesion may be identified as a training medical image with a high risk of occurrence of the lesion. Examples of the training medical image 530 of a non-lesioned region of a patient having the lesion may include a training medical image of the left lung of a patient having lung cancer in the right lung, a training medical image of the left kidney of a patient having kidney cancer in the right kidney, a training medical image of the left foot of a patient having a specific lesion on the right foot, and the like. The low-risk group training medical image may include a training medical image 540 of a target region of a patient not having the lesion.

As a specific example, an example of a training medical image for predicting the risk of occurrence of breast cancer is illustrated in FIG. 5. The training medical image for predicting the risk of occurrence of breast cancer may include a mammography image 510 of a cancer region of a patient diagnosed with breast cancer, a mammography image 520 of the breasts of the patient diagnosed with breast cancer, which was obtained before the patient was diagnosed with breast cancer, a mammography image 530 of the opposite breast of the patient having breast cancer in one breast, and mammography images 540 of breasts of patients who have never been diagnosed with breast cancer. In this case, the mammography image 510 of the patient diagnosed with breast cancer, the mammography image 520 of the breasts of the patient diagnosed with breast cancer before the patient was diagnosed with breast cancer, and the mammography image 530 of the opposite breast of the patient having breast cancer in one breast may be included in the high-risk group training medical image, and the mammography images 540 of breasts of patients who have never been diagnosed with breast cancer may be included in the low-risk group training medical image.

Additionally, the training data may further include information on lesions associated with the high-risk group training medical image. The information on lesions associated with the high-risk group training medical image may be included in the high-risk group training medical image as the mask annotation information labeled in pixel level. This information may be used to infer a region in the received medical image where the lesion is expected to occur. For example, in the example illustrated in FIG. 5, the mammography image 510 of the patient diagnosed with breast cancer may further include mask annotation information in which a region 512 with occurrence of cancer is labeled in pixel level. As another example, in the example illustrated in FIG. 5, the mammography image 520 of the breast of the patient diagnosed with breast cancer before they were diagnosed with breast cancer may further include mask annotation information in which a region 522 with occurrence of cancer is labeled in pixel level after the patient is diagnosed with breast cancer.

Each training medical image may include a plurality of sub training medical images. For example, in the example illustrated in FIG. 5, each of the training medical images 510, 520, 530, and 540 may include two craniocaudal (CC) images and two medial lateral oblique (MLO) images.

Additionally, the training data may further include training additional information related to the risk of occurrence of the lesion of each reference patient. For example, the training additional information may include clinical data, lab data, and/or biological data of each patient. As a specific example, for the prediction on a risk of occurrence of breast cancer, the training additional information may include at least one of the age, weight, family history, height, gender, age at menarche, menopausal status, birth history, hormone replacement therapy treatment history, genetic information of a reference patient (e.g., BRCA, BRD, PTEN, TP53, CDH1, SKT11/LKB 1, PALB2, and the like), and breast density.

In the training medical images, the number of high-risk group training medical images and low-risk group training medical images may not be balanced. In this case, the information processing system may balance training through tasks such as processing at least some of the training medical images or adjusting training weights. For example, if there are significantly more low-risk group training medical images than the high-risk group training medical images, the machine learning model may not be able to classify the high-risk group well, which may degrade the performance of the model. In this case, for the training, the information processing system may process the high-risk group training medical images to increase the number of high-risk group training medical images (over sampling), to decrease the number of low-risk group training medical images (under sampling), or to use the two methods described above at the same time (hybrid sampling), or may adjust the training weight.

FIG. 6 is a diagram illustrating an example of a machine learning model 620. As illustrated, the machine learning model 620 may output a prediction result 630 on a risk of occurrence of a lesion based on a medical image 610. The prediction result 630 on a risk of occurrence of a lesion may be output as information in which the risk of occurrence of the lesion is expressed by a means (e.g., score, probability, color, and the like) that can express the degree of severity of the risk, information that is classified into a plurality of classes (high risk, intermediate risk, low risk, and the like) according to the degree of risk of occurrence of the lesion, etc.

The machine learning model 620 may receive a plurality of training medical images and be trained to infer a reference prediction result on a risk of occurrence of the lesion. For example, in order to generate and train the machine learning model 620, the processor (e.g., 220 of FIG. 2) may receive a plurality of training medical images and reference prediction results associated with the plurality of training medical images. When training the machine learning model 620, the processor may use information on the reference prediction results associated with a plurality of training medical images as ground truth.

Additionally, the processor may further receive information of the lesion associated with the training medical image so as to generate and train the machine learning model 620. The information on lesions associated with the training medical image may be included in the training medical image as the mask annotation information labeled in pixel level. This information may be used to infer a region in the received medical image where a lesion is expected to occur. For example, in the received medical image, the processor may output a region where cancer is expected to occur in a specific color, output a boundary of the region where cancer is expected to occur, or output a heat map or the like in which each pixel is expressed in color according to the degree that cancer is expected to occur. All information may be included in the prediction result 630 on the risk of occurrence of the lesion.

FIG. 7 a diagram illustrating an example of a machine learning model 720. The processor may use the machine learning model 720 to classify a plurality of training medical images 710 into a plurality of classes so as to generate or train the machine learning model 720 that outputs a prediction result on a risk of occurrence of a lesion of each patient. The processor may learn the training medical images classified to correspond to a plurality of classes. For example, the machine learning model 720 may include one or more classifiers, and may be trained to output a classification result 730 of classifying the plurality of training medical images 710 into a plurality of classes.

For example, the processor may train the machine learning model 720 to classify the plurality of training medical images 710 into either the high-risk group training medical images or the low-risk group training medical images. As another example, the processor may train the machine learning model 720 to classify the plurality of training medical images 710 into one of a training medical image 732 of a lesion region of a patient having the lesion, a training medical image 734 of the lesion region of a patient having the lesion, which was obtained before occurrence of the lesion in lesion region, a training medical image 736 of a non-lesioned region of a patient having the lesion, or a training medical image 738 of a patient not having a history of lesion occurrence.

In FIG. 7, the machine learning model 720 is illustrated as including one classifier, but is not limited thereto. For example, the machine learning model may include a plurality of classifiers as illustrated in FIG. 8.

FIG. 8 is a diagram illustrating an example of training a machine learning model 820. The processor may train the machine learning model 820 to output a classification result 830 of classifying a plurality of training medical images 810 into a plurality of classes so as to generate or train the machine learning model 820 that outputs a prediction result on a risk of occurrence of a lesion of a patient. For example, the machine learning model 820 may include a plurality of classifiers 822, 824, and 826, and the processor may train the machine learning model 820 such that the training medical images 810 are classified into a plurality of classes through at least one of the plurality of classifiers 822, 824, and 826.

The machine learning model 820 may include a first classifier 822 that classifies the training medical images 810 into a first class and remaining classes, a second classifier 824 that classifies the training medical images 810 into a second class and other classes, and a third classifier 826 that classifies the training medical images 810 into a third class and other classes. In this case, the processor may train the machine learning model 820 such that the training medical images 810 are classified into one of the first class, the second class, the third class, and the fourth class through at least one of the plurality of classifiers 822, 824, and 826 included in the machine learning model 820.

The machine learning model 820 may include the first classifier 822 that classifies the training medical images 810 into a training medical image of a lesion region of a patient having the lesion, and the rest training medical images, the second classifier 824 that classifies the training medical images 810 into a training medical image of the lesion region of a patient having the lesion, which was obtained before occurrence of the lesion in lesion region, and the rest of the training medical images, and the third classifier 826 that classifies the training medical images 810 into a training medical image of a non-lesioned region of a patient having the lesion, and the rest training medical images. The machine learning model 820 may be trained to classify at least one of the training medical image of the lesion region of a patient, the training medical image of the lesion region of a patient, which was obtained before occurrence of the lesion in the lesion region, or the training medical image of a non-lesioned region of a patient having the lesion into a high-risk group, and classify the training medical image of a patient without the lesion into a low-risk group.

In this case, the processor may train the machine learning model 820 such that the training medical images 810 are classified into one of a training medical image of the lesion region of a patient having the lesion, a training medical image of the lesion region of a patient having the lesion which was obtained before occurrence of the lesion in lesion region, a training medical image of a non-lesioned region of a patient having the lesion, or a training medical image of a patient not having a history of lesion occurrence, through at least one of the plurality of classifiers 822, 824, and 826 included in the machine learning model 820.

The processor may train the machine learning model 820 to classify the training medical images 810 hierarchically. For example, the machine learning model 820 may include the first classifier 822 that detects all classes other than the first class in the training medical images 810, the second classifier 824 that detects all classes other than the second class in the training medical images detected by the first classifier 822, and the third classifier 826 that detects all classes other than the third class in the training medical images detected by the second classifier 824. In this case, the processor may train the machine learning model 820 such that the training medical images 810 are classified into one of the first class, the second class, the third class, or the fourth class by sequentially applying at least one classifier.

As a specific example, the machine learning model 820 may include the first classifier 822 that detects, in the training medical images 810, all training medical images other than the training medical images of patients without a history of lesion occurrence, the second classifier 824 that detects, in the training medical images detected by the first classifier 822, all training medical images other than the training medical images of a non-lesioned region of patients having the lesion, and the third classifier 826 that detects, in the training medical images detected by the second classifier 824, all training medical images other than the training medical images of a lesion region of a patient having the lesion, which was obtained before occurrence of the lesion in the lesion region. In this case, the processor may train the machine learning model 820 such that the training medical images 810 are classified into one of a training medical image of a lesion region of a patient having the lesion, a training medical image of the lesion region of a patient having the lesion, which was obtained before occurrence of the lesion in the lesion region, a training medical image of a non-lesioned region of a patient having the lesion, or a training medical image of a patient not having a history of lesion occurrence, through at least one of the plurality of classifiers 822, 824, and 826 included in the machine learning model 820 in sequence.

As such, using the machine learning model 820 including the plurality of classifiers, it is possible to classify the degree of the risk of occurrence of the lesion more accurately based on the medical images of patients, thereby providing a prediction result more accurately.

FIG. 9 is a diagram illustrating an example in which a machine learning model 920 outputs a prediction result 940 on a risk of occurrence of a lesion based on a plurality of sub medical images 912, 914, 916, and 918. The medical image taken of one subject may include a plurality of sub medical images. For example, a medical image of the breast captured by mammography for diagnosing breast cancer may include a total of four sub medical images including medial lateral oblique (MLO) images and craniocaudal (CC) images of each of the breasts on both sides.

The processor may output the prediction result 940 on the risk of occurrence of the lesion based on a medical image 910 using the machine learning model 920, in which the medical image 910 may include a plurality of sub medical images 912, 914, 916, and 918. For example, the medical image 910 may include a plurality of sub medical images 912, 914, 916, and 918 of a target region where a target disease may occur at various positions or from various angles. As a specific example, in case of predicting the risk of occurrence of breast cancer, the medical image 910 may include a mammography image, and the plurality of sub medical images may include two craniocaudal (CC) images and two medial lateral oblique (MLO) images. In addition, the machine learning model 920 may be a convolutional neural network (CNN) model, for example.

If the medical image 910 includes a plurality of sub medical images 912, 914, 916, and 918, the processor may input the plurality of sub medical images 912, 914, 916, and 918 to the machine learning model 920 and extract a plurality of feature maps 932, 934, 936, and 938 output for each of the plurality of sub medical images 912, 914, 916, and 918 from at least one layer (e.g., intermediate layer or output layer, and the like) included in the machine learning model 920, and aggregate the plurality of extracted feature maps 932, 934, 936, and 938 and output the prediction result 940 on the risk of occurrence of the lesion. For example, the processor may input a plurality of sub medical images 912, 914, 916, and 918 to the machine learning model and aggregate the plurality of feature maps 932, 934, 936, and 938 by concatenating or summing each of the plurality of feature maps 932, 934, 936, and 938 output from the middle layer of the machine learning model 920, and output the prediction result 940 on the risk of occurrence of the lesion by using the plurality of aggregated feature maps.

As another example, the processor may input a plurality of sub medical images 912, 914, 916, and 918 to the machine learning model 920 and apply a weight to a specific region in each of the plurality of feature maps 932, 934, 936, and 938 output from the middle layer of the machine learning model 920, and output the prediction result 940 on the risk of occurrence of the lesion. Specifically, the processor may pass the plurality of feature maps 932, 934, 936, and 938 output from at least one layer included in the machine learning model 920 through an attention module or a transformer module, and focus on a more important part (e.g., a feature map output based on a specific sub medical image or a specific part of a feature map output based on a specific pixel region, and the like) for inferring a prediction result in the plurality of feature maps 932, 934, 936, and 938 so as to output the prediction result 940 on the risk of occurrence of the lesion. This attention module or transformer module may be included in the machine learning model 920 or may be a module or network connected to the machine learning model 920.

As described above, by outputting the prediction result 940 on a risk of occurrence of the lesion based on the plurality of sub medical images 912, 914, 916, and 918, it is possible to provide a more accurate prediction result, and in particular, by focusing on a part that is more important in generating a prediction result, it is possible to further increase the accuracy of prediction.

FIG. 10 is a diagram illustrating an example of generating a prediction result 1040 on a risk of occurrence of a lesion based on a medical image 1010 and additional information 1020. The processor may further receive not only the medical image 1010 of the patient, but also the additional information 1020 of the patient related to the risk of occurrence of the lesion so as to generate the prediction result 1040 on the risk of occurrence of the lesion of the patient. In this case, the additional information 1020 may include clinical data, lab data, and/or biological data. As a specific example, for the prediction on a risk of occurrence of breast cancer, the additional information 1020 may include at least one of the age, weight, family history, height, gender, age at menarche, menopausal status, birth history, hormone replacement therapy treatment history, genetic information of a patient (e.g., BRCA, BRD, PTEN, TP53, CDH1, SKT11/LKB1, PALB2, and the like), and breast density.

The processor may use the received medical image 1010 and additional information 1020 to output the prediction result 1040 on the risk of occurrence of the lesion. For example, using a machine learning model 1030 trained to output a reference prediction result on a risk of occurrence of the lesion based on a plurality of training medical images and training additional information, the processor may output the prediction result 1040 on the risk of occurrence of the lesion based on the received medical image 1010 and additional information 1020.

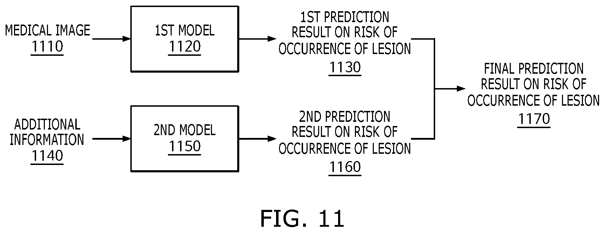

FIG. 11 is a diagram illustrating an example of generating a final prediction result 1170 on a risk of occurrence of a lesion based on a medical image 1110 and additional information 1140. The processor may use a plurality of models 1120 and 1050 to output the final prediction result 1170 on the risk of occurrence of the lesion based on the received medical image 1110 and additional information 1140. For example, the processor may use the first model 1120, which is trained to output a reference prediction result on a risk of occurrence of the lesion based on each training medical images, to output a first prediction result 1130 on a risk of occurrence of the lesion based on the medical image 1110. In addition, the processor uses a second model 1150, which is trained to output a reference prediction result on a risk of occurrence of the lesion based on the training additional information, to output a second prediction result 1160 on a risk of occurrence of the lesion based on the additional information 1140. The processor may output the final prediction result 1170 on the risk of occurrence of the lesion by using the first prediction result 1130 and the second prediction result 1160.

FIGS. 10 to 11 merely show an example of a configuration of a model for generating a prediction result based on a medical image and additional information, but another implementation is possible. For example, a model having any configuration capable of generating a prediction result based on a medical image and additional information may be used. As a specific example, at least one of the illustrated models 1030, 1120, and 1150 may be any algorithm other than a machine learning model. As another specific example, in the example illustrated in FIG. 11, the second model 1150 may be configured to receive not only the additional information 1140, but also the first prediction result 1130 (or information obtained by processing the first prediction result 1130) on the risk of occurrence of the lesion output by the additional information 1140 and the first model 1120, and output the final prediction result 1170 on a risk of occurrence of the lesion based on the additional information 1140 and the first prediction result 1130 on the risk of occurrence of the lesion output by the first model 1120.

In this way, the accuracy of prediction may be further improved by predicting the risk of occurrence of the lesion in consideration of not only the medical image but also the additional information of the patient.

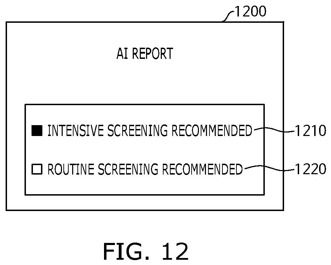

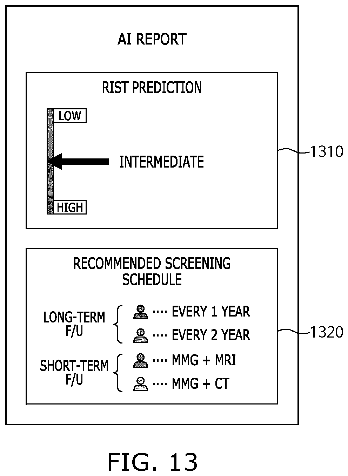

FIGS. 12 and 13 are diagrams illustrating examples of providing a prediction result 1310 and medical information 1200 and 1320 based on the prediction result. The information processing system may output a prediction result on a risk of occurrence of a lesion. Additionally or alternatively, the information processing system may output information related to at least one of medical examination, diagnosis, prevention, or treatment based on the prediction result on a risk of occurrence of the lesion. For example, the information processing system may provide a prediction result on a risk of occurrence of the lesion of a patient and/or various medical information generated based on the prediction result to the user terminal. In addition, the user terminal may receive the prediction result on the risk of occurrence of the lesion of the patient and/or various medical information generated based on the prediction result from the information processing system and output the received information through a display device.

The prediction result on the risk of occurrence of the lesion may include information in which the risk of occurrence of the lesion is expressed by a means (such as a numerical value or color) that can express the degree of severity of the risk, information that is classified into a plurality of classes (e.g., high risk, intermediate risk, low risk) according to the degree of risk of occurrence of the lesion, etc.

The medical information based on the prediction result on a risk of occurrence of the lesion may include information on the prognosis of a patient, a necessary intervention (e.g., treatment, diagnosis, test, prevention policy and timing) required of the patient in a specific situation, drug response, or the like. For example, the medical information may include a personalized screening schedule according to the degree of risk of occurrence of the lesion. As a specific example, it may recommend additional examinations (e.g., MRI, CT scans, or the like) to a patient with a high risk of occurrence of the lesion, and provide a screening schedule for intensive screening at short intervals. On the other hand, a patient with a low risk of occurrence of the lesion may be provided with a screening schedule for routine screening at long intervals.

FIG. 12 illustrates an example in which medical information 1200 is output based on a prediction result. As illustrated in FIG. 12, the medical information may include necessary intervention according to the degree of risk of occurrence of the lesion. The intensive screening may be recommended for a patient with a high risk of occurrence of the lesion, and the routine screening may be recommended for a patient with a low risk of occurrence of the lesion.

FIG. 13 illustrates an example in which the prediction result 1310 and the medical information 1320 based on the prediction result are output. As illustrated in FIG. 13, the information processing system may classify the prediction result 1310 into a plurality of classes (high risk, intermediate risk, and low risk) according to the degree of risk of occurrence of a lesion and output the classified result. For example, as illustrated, a prediction result of "Intermediate" may be output for a medical image of a patient having a moderate risk of occurrence of the lesion. Additionally, the information processing system may output the medical information 1320 based on the prediction result. For example, the information processing system may output a personalized screening schedule 1320 according to the degree of risk of occurrence of the lesion. As a specific example, as illustrated, a screening schedule for routine screening at long intervals (e.g., 1 year, 2 years or the like) may be output for a patient having a relatively low risk of occurrence of the lesion. On the other hand, for a patient with a relatively high risk of occurrence of the lesion, an additional screening (e.g., MRI or CT scan, and the like) may be recommended, and a screening schedule for intensive screening at a short interval may be output.

As described above, since the information on appropriate interventions, schedules, and the like related to treatment, diagnosis, screening, prevention is provided according to the prediction result on the risk and/or degree of severity of the risk of occurrence of the lesion of each patient, the medical personnel provided with the information can efficiently and effectively manage limited resources (e.g., personnel, equipment, pharmaceuticals, and the like). Furthermore, on the patient's side, a high-risk group patient provided with the information can prevent disease or detect disease early through additional screening or short interval screening, and a low-risk group patient provided with the information can save money and time through long interval screening or the like.

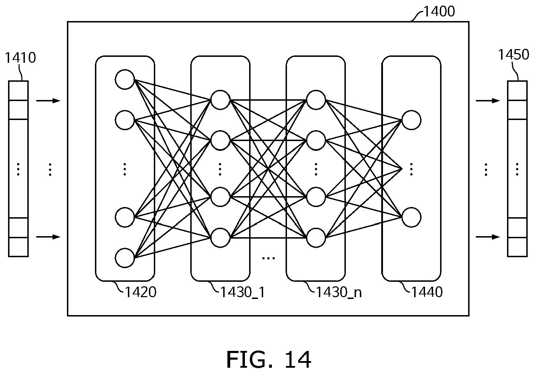

FIG. 14 is an exemplary diagram illustrating an artificial neural network model 1400. In machine learning technology and cognitive science, the artificial neural network model 1400 as an example of the machine learning model refers to a statistical learning algorithm implemented based on a structure of a biological neural network, or to a structure that executes such algorithm.

The artificial neural network model 1400 may represent a machine learning model that acquires a problem solving ability by repeatedly adjusting the weights of synapses by the nodes that are artificial neurons forming the network through synaptic combinations as in the biological neural networks, thus training to reduce errors between a target output corresponding to a specific input and a deduced output. For example, the artificial neural network model 1400 may include any probability model, neural network model, and the like, which is used in artificial intelligence learning methods such as machine learning and deep learning.

The artificial neural network model 1400 may include an artificial neural network model configured to predict a risk of occurrence of a lesion of a patient (e.g., to generate information on a prediction result) based on an input medical image of the patient. Additionally or alternatively, the artificial neural network model 1400 may include an artificial neural network model configured to predict a risk of occurrence of the lesion of a patient based on input additional information of the patient. Additionally or alternatively, the artificial neural network model 1400 may include an artificial neural network model configured to predict a risk of occurrence of the lesion of a patient based on an input medical image of the patient and additional information of the patient. Additionally or alternatively, the input medical image of the patient may include a plurality of sub medical images, and the artificial neural network model 1400 may include an artificial neural network model configured to predict a risk of occurrence of the lesion of a patient based on a plurality of input sub medical images and/or additional information of the patient.

The artificial neural network model 1400 is implemented as a multilayer perceptron (MLP) formed of multiple nodes and connections between them. The artificial neural network model 1400 may be implemented using one of various artificial neural network model structures including the MLP. As illustrated in FIG. 14, the artificial neural network model 1400 includes an input layer 1420 to receive an input signal or data 1410 from the outside, an output layer 1440 to output an output signal or data 1450 corresponding to the input data, and (n) number of hidden layers 1430_1 to 1430_n (where n is a positive integer) positioned between the input layer 1420 and the output layer 1440 to receive a signal from the input layer 1420, extract the features, and transmit the features to the output layer 1440. In an example, the output layer 1440 receives signals from the hidden layers 1430_1 to 1430_n and outputs them to the outside.

The method of training the artificial neural network model 1400 includes the supervised learning that trains to optimize for solving a problem with inputs of teacher signals (correct answers), and the unsupervised learning that does not require a teacher signal. The information processing system may train the artificial neural network model 1400 by supervised learning and/or unsupervised learning to generate information related to a prediction result on a risk of occurrence of the lesion of a patient based on the medical image of the patient. For example, the information processing system may train the artificial neural network model 1400 by supervised learning to generate reference information related to a reference prediction result of each of reference patients based on each training medical image of each reference patient.

In another example, the information processing system may train the artificial neural network model 1400 by supervised learning and/or unsupervised learning to generate information related to prediction results on a risk of occurrence of the lesion based on the additional information of each of the reference patients. For example, the information processing system may train the artificial neural network model 1400 by supervised learning to generate reference information related to reference prediction results of the reference patients based on the training additional information of the reference patients.

In still another example, the information processing system may train the artificial neural network model 1400 by supervised learning and/or unsupervised learning to generate information related to prediction results on a risk of occurrence of the lesion based on each of the medical images of the reference patients and the additional information of the reference patients. For example, the information processing system may train the artificial neural network model 1400 by supervised learning to generate reference information related to a reference prediction result of the reference patient based on the medical image of the reference patient and the training additional information of the reference patient.

In still another example, the medical image of the reference patient may include a plurality of sub medical images, and the information processing system may train the artificial neural network model 1400 by supervised learning and/or unsupervised learning to generate information related to a prediction result on a risk of occurrence of the lesion based on a plurality of sub medical images and/or the additional information of each of the reference patients. For example, the information processing system may train the artificial neural network model 1400 by supervised learning to generate reference information related to reference prediction results on the reference patients based on the plurality of sub training medical images of the reference patients and/or the training additional information of the reference patients.

The artificial neural network model 1400 trained as described above may be stored in a memory (not illustrated) of the information processing system and may predict the risk of occurrence of the lesion of a patient in response to inputting a medical image of the patient received from the communication module and/or the memory, so as to generate a result of prediction on a risk of occurrence of the lesion of a patient. Additionally or alternatively, the artificial neural network model 1400 may predict the risk of occurrence of the lesion of a patient in response to inputting additional information of the patient, so as to generate a prediction result on the risk of occurrence of the lesion of the patient. Additionally or alternatively, the artificial neural network model 1400 may predict the risk of occurrence of the lesion of a patient in response to inputting the medical image of the patient and the additional information of the patient, so as to generate a prediction result on a risk of occurrence of the lesion of the patient.

The input variables to the artificial neural network model that generates information on a prediction result on a risk of occurrence of a lesion of a patient may be the medical image of the patient and/or the additional information of the patient. For example, the input variables input to the input layer 1420 of the artificial neural network model 1400 may be an image vector 1410 including a medical image of a patient as one vector data element and/or a vector 1410 including additional information of the patient as one vector data element. In response to these inputs, an output variable output from the output layer 1440 of the artificial neural network model 1400 may be a vector 1450 representing or characterizing information on a prediction result on a risk of occurrence of the lesion of the patient. That is, the output layer 1440 of the artificial neural network model 1400 may be configured to output a vector representing or characterizing information related to the prediction result on the risk of occurrence of the lesion of the patient. In the present disclosure, the output variable of the artificial neural network model 1400 is not limited to the types described above, and may include any information/data representing information on the prediction result on the risk of occurrence of the lesion of the patient. In addition, the output layer 1440 of the artificial neural network model 1400 may be configured to output a vector indicating reliability and/or accuracy of information, etc. related to the prediction result on the risk of occurrence of the lesion of the patient.

As described above, the input layer 1420 and the output layer 1440 of the artificial neural network model 1400 are respectively matched with a plurality of output variables corresponding to a plurality of input variables, and as the synaptic values between nodes included in the input layer 1420, and the hidden layers 1430_1 to 1430_n, and the output layer 1440 are adjusted, training can be processed to extract a correct output corresponding to a specific input. Through this training process, the features hidden in the input variables of the artificial neural network model 1400 may be confirmed, and the synaptic values (or weights) between the nodes of the artificial neural network model 1400 may be adjusted so as to reduce the errors between the output variable calculated based on the input variable and the target output. The artificial neural network model 1400 trained as described above may output information related to a prediction result on a risk of occurrence of the lesion of a patient, in response to inputting a medical image of the patient and/or additional information of the patient.

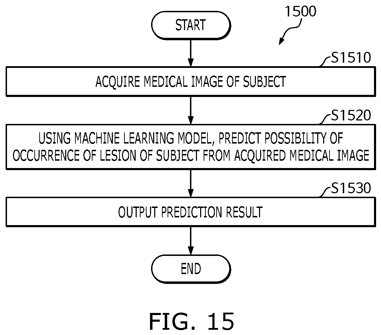

FIG. 15 is a flow diagram illustrating an example of a method 1500 for predicting the risk of occurrence of a lesion. The method 1500 may be initiated by a processor (e.g., the information processing system or one or more processors of a user terminal) acquiring a medical image of a subject, at S 1510. In this case, the subject may refer to a region that is a target for predicting the risk of occurrence of the lesion. Acquiring an image of the subject may include receiving a medical image from an external device (user terminal, medical diagnosis device, and the like), receiving a medical image from a server, acquiring a medical image stored in an internal memory, and the like.

The medical image may include a plurality of sub medical images. For example, the medical image may include a mammography image, and the plurality of sub medical images may include two craniocaudal (CC) images and two medial lateral oblique (MLO) images.

Additionally, the processor may further receive additional information related to the risk of occurrence of the lesion. In this case, the additional information may include clinical data, lab data and/or biological data. As a specific example, for the prediction on a risk of occurrence of breast cancer, the additional information may include at least one of the age, weight, family history, height, gender, age at menarche, menopausal status, birth history, hormone replacement therapy treatment history, genetic information of a patient (e.g., BRCA, BRD, PTEN, TP53, CDH1, SKT11/LKB 1, PALB2, and the like), and breast density.

The processor may predict the possibility of occurrence of the lesion of the subject from the acquired medical image by using the machine learning model, at S 1520. In this case, the machine learning model is a model trained with a plurality of training medical images and a risk of occurrence of the lesion associated with each of the plurality of training medical images.

The plurality of training medical images may include a high-risk group training medical image and a low-risk group training medical image, and the high-risk group training medical image may be classified into a plurality of classes according to the degree of risk of occurrence of the lesion. For example, the high-risk group training medical image may include at least one of a training medical image of a lesion region of each of patients having the lesion, a training medical image of a lesion region of each patient having the lesion, which was obtained before occurrence of the lesion in lesion region, or a training medical image of a non-lesioned region of each patient having the lesion. The non-lesioned region of the patient having the lesion may include at least one of a region opposite to the lesion region or a region surrounding the lesion region.

A machine learning model may include one or more classifiers. For example, the machine learning model may include a first classifier trained to classify the plurality of training medical images into a high-risk group training medical image or a low-risk group training medical image, and a second classifier trained to classify the classified high-risk group training medical images into a plurality of classes.

Additionally, the machine learning model may be a model further trained to infer the mask annotation information in the high-risk group training medical images from the high-risk group training medical images. In this case, the processor may use a machine learning model to output a region (e.g., one or more pixel regions) in which the lesion is expected to occur in the acquired medical image.

If the medical image includes a plurality of sub medical images, the processor may input a plurality of sub medical images to the machine learning model and extract a plurality of feature maps output from at least one layer included in the machine learning model, and aggregate a plurality of extracted feature maps and use the aggregated plurality of feature maps to output a prediction result on a risk of occurrence of the lesion. For example, the processor may input a plurality of sub medical images to the machine learning model and concatenate or sum a plurality of feature maps output from at least one layer included in the machine learning model to aggregate a plurality of extracted feature maps and use the aggregated plurality of feature maps and output a prediction result on a risk of occurrence of the lesion. As another example, the processor may input a plurality of sub medical images to the machine learning model and apply a weight to a specific region included in each of a plurality of feature maps output from at least one layer included in the machine learning model, and output prediction result on a risk of occurrence of the lesion. Specifically, the processor may pass a plurality of feature maps output from at least one layer included in the machine learning model through the attention layer or transformer attention layer, and focus on a part (e.g., a feature map output based on a specific pixel region or a specific sub medical image) that is more important for inferring a prediction result among the plurality of feature maps, and output the prediction result on a risk of occurrence of the lesion.

Additionally or alternatively, the processor may use a machine learning model to output a prediction result on a risk of occurrence of the lesion based on the acquired medical image and the received additional information. For example, the processor may use a machine learning model further trained to output a reference prediction result on a risk of occurrence of the lesion based on a plurality of training medical images and training additional information, and output the prediction result on a risk of occurrence of the lesion based on the acquired medical image and the additional information. As another example, the processor may use a machine learning model to output a first prediction result on a risk of occurrence of the lesion based on the acquired medical image, use an additional machine learning model to output a second prediction result on a risk of occurrence of the lesion based on the additional information, and use the first prediction result and the second prediction result to generate a final prediction result on the risk of occurrence of the lesion. In this example, the additional machine learning model may be a model trained to output a reference prediction result on a risk of occurrence of the lesion based on the training additional information.

The processor may output the prediction result, at S1530. In this example, the outputting the prediction result may include at least one of transmitting an image representing the prediction result to an external display device, delivering a report including the prediction result to the user terminal, uploading the prediction result to the server, and directly displaying it to the user using a display device connected to the information processing system.

The processor may provide information related to at least one of medical examination, diagnosis, prevention, or treatment based on the prediction result on a risk of occurrence of the lesion. For example, the information related to at least one of medical examination, diagnosis, prevention, or treatment may provide prognosis of a patient, necessary intervention (e.g., treatment, diagnosis, examination, prevention policy and timing) required of a patient in a specific situation, or information on drug response and the like. As a specific example, the processor may provide a personalized screening schedule according to the degree of risk of occurrence of the lesion. The processor may recommend additional examinations (e.g., MRI, CT scans, or the like) to a patient with a high risk of occurrence of the lesion, and may provide a screening schedule for routine screening at short intervals. On the other hand, a patient with a low risk of occurrence of the lesion may be provided with a screening schedule for routine screening at long intervals.

The flowchart illustrated in FIG. 15 and the above description are merely examples, and may be implemented in various ways. For example, one or more steps may be added or omitted, the order of each step may be changed, or at least some steps may be performed overlapping.

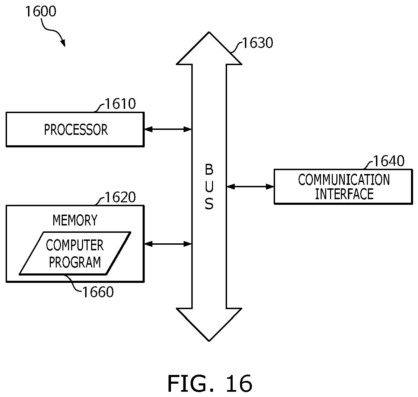

FIG. 16 illustrates an exemplary configuration of a system for predicting a risk of occurrence of a lesion. An information processing system 1600 of FIG. 16 may be an example of the information processing system 100 described with reference to FIG. 2. As illustrated, the information processing system 1600 includes one or more processors 1610, a bus 1630, a communication interface 1640, and a memory 1620 for loading a computer program 1660 executed by the processor 1610. Meanwhile, only the components related to the present example are illustrated in FIG. 16. Accordingly, those of ordinary skill in the art to which the present disclosure pertains will be able to recognize that other general-purpose components may be further included in addition to the components illustrated in FIG. 16.

The processors 1610 control the overall operation of components of the information processing system (e.g., the information processing system 100). In present disclosure, the processor 1610 may be configured with a plurality of processors. The processor 1610 may include central processing unit (CPU), micro processor unit (MPU), micro controller unit (MCU), graphic processing unit (GPU), field programmable gate array (FPGA), at least two of any types of processors well known in the technical field of the present disclosure. In addition, the processor 1610 may perform computation on at least one application or program for executing the method according to various examples.

The memory 1620 may store various types of data, instructions, and/or information. The memory 1620 may load one or more computer programs 1660 in order to execute the method/operation according to various examples. The memory 1620 may be implemented as a volatile memory such as RAM, but the technical scope of the present disclosure is not limited thereto. For example, the memory 1620 may include a nonvolatile memory such as a read only memory (ROM), an erasable programmable ROM (EPROM), an electrically erasable programmable ROM (EEPROM), a flash memory, and the like, a hard disk, a detachable disk, or any type of computer-readable recording medium well known in the art to which the present disclosure pertains.

The bus 1630 may provide a communication function between components of the information processing system. The bus 1630 may be implemented as various types of buses such as an address bus, a data bus, a control bus, or the like.

The communication interface 1640 may support wired/wireless Internet communication of the information processing system. In addition, the communication interface 1640 may support various other communication methods in addition to the Internet communication. To this end, the communication interface 1640 may include a communication module well known in the technical field of the present disclosure.

The computer program 1660 may include one or more instructions that cause the processors 1610 to perform operations/methods in accordance with various examples. That is, the processors 1610 may execute the one or more instructions so as to perform operations/methods according to various examples.

For example, the computer program 1660 may include one or more instructions for performing an operation of receiving a medical image and an operation of outputting a prediction result on a risk of occurrence of the lesion based on the received medical image by using a machine learning model. In this case, a system for predicting the risk of occurrence of the lesion may be implemented through the information processing system 1600.

The above description of the present disclosure is provided to enable those skilled in the art to make or use the present disclosure. Various modifications of the present disclosure will be readily apparent to those skilled in the art, and the general principles defined herein may be applied to various modifications without departing from the spirit or scope of the present disclosure. Thus, the present disclosure is not intended to be limited to the examples described herein but is intended to be accorded the broadest scope consistent with the principles and novel features disclosed herein.

Although example implementations may refer to utilizing aspects of the presently disclosed subject matter in the context of one or more standalone computer systems, the subject matter is not so limited, and they may be implemented in conjunction with any computing environment, such as a network or distributed computing environment. Furthermore, aspects of the presently disclosed subject matter may be implemented in or across a plurality of processing chips or devices, and storage may be similarly influenced across a plurality of devices. Such devices may include PCs, network servers, and handheld devices.

Although the present disclosure has been described in connection with certain examples herein, it should be understood that various modifications and changes can be made without departing from the scope of the present disclosure, which can be understood by those skilled in the art to which the present disclosure pertains. In addition, such modifications and changes should be considered within the scope of the claims appended herein.

## Claims

1. A method performed by one or more processors to predict a risk of occurrence of a lesion, the method comprising:
acquiring a medical image of a subject;
by using a machine learning model, predicting a possibility of occurrence of a lesion of the subject based on the acquired medical image; and
outputting a result of the predicting,
wherein the machine learning model is a model trained with a plurality of training medical images and a risk of occurrence of the lesion associated with each of the plurality of training medical images.

2. The method according to claim 1, wherein the plurality of training medical images include a high-risk group training medical image and a low-risk group training medical image, and
the high-risk group training medical image includes a first training medical image of a lesion region of a patient having the lesion, which was obtained before occurrence of the lesion in the lesion region.

3. The method according to claim 1, wherein the plurality of training medical images include a high-risk group training medical image and a low-risk group training medical image, and
the high-risk group training medical image includes a second training medical image of a non-lesioned region of a patient having the lesion.

4. The method according to claim 3, wherein the non-lesioned region of the patient includes at least one of a region opposite to the lesion region or a region surrounding the lesion region.

5. The method according to claim 1, wherein the plurality of training medical images are classified into a plurality of classes according to a degree of risk of occurrence of the lesion.

6. The method according to claim 1, wherein the machine learning model includes:
a first classifier trained to classify the plurality of training medical images into a high-risk group training medical image or a low-risk group training medical image; and
a second classifier trained to classify the classified high-risk group training medical images into a plurality of classes.

7. The method according to claim 1, wherein the machine learning model is a model that is further trained to infer mask annotation information in the training medical images from the training medical images, and
the predicting the possibility of occurrence of the lesion includes, by using the machine learning model, outputting a region in which the lesion is expected to occur in the acquired medical image.

8. The method according to claim 1, wherein the medical image includes a plurality of sub medical images, and
the predicting the possibility of occurrence of the lesion includes:
extracting a plurality of feature maps output from at least one layer included in the machine learning model by inputting the plurality of sub medical images to the machine learning model;
aggregating the plurality of extracted feature maps; and
outputting a prediction result on a risk of occurrence of the lesion based on the aggregated plurality of feature maps.

9. The method according to claim 8, wherein the aggregating the plurality of extracted feature maps includes concatenating or summing the plurality of feature maps.

10. The method according to claim 8, wherein the outputting the prediction result on the risk of occurrence of the lesion by using the aggregated plurality of feature maps includes outputting the prediction result on the risk of occurrence of the lesion by applying a weight to a specific region within each of the plurality of feature maps.

11. The method according to claim 8, wherein the medical image includes a mammography image, and
the plurality of sub medical images include two craniocaudal (CC) images and two medial lateral oblique (MLO) images.

12. The method according to claim 1, further comprising receiving additional information related to the risk of occurrence of the lesion, wherein
the predicting the possibility of occurrence of the lesion includes, by using the machine learning model, outputting a prediction result on the risk of occurrence of the lesion based on the acquired medical image and the additional information.

13. The method according to claim 12, wherein the machine learning model is a model that is further trained to output a reference prediction result on the risk of occurrence of the lesion based on the plurality of training medical images and training additional information.

14. The method according to claim 1, further comprising receiving additional information related to a risk of occurrence of the lesion,
wherein the predicting the possibility of occurrence of the lesion includes:
by using the machine learning model, outputting a first prediction result on the risk of occurrence of the lesion based on the acquired medical image;
by using an additional machine learning model, outputting a second prediction result on the risk of occurrence of the lesion based on the additional information; and
generating a final prediction result on the risk of occurrence of the lesion based on the first prediction result and the second prediction result, and
wherein the additional machine learning model is a model trained to output a reference prediction result on the risk of occurrence of the lesion based on training additional information.

15. The method according to claim 1, wherein the outputting the result of the predicting includes outputting information related to at least one of medical examination, diagnosis, prevention or treatment, based on the result of the predicting.

16. A non-transitory computer-readable recording medium storing instructions that, when executed by one or more processors, cause performance of the method according to claim 1.

17. An information processing system comprising:
a memory; and
one or more processors connected to the memory and configured to execute one or more computer-readable programs included in the memory, wherein the one or more programs include instructions for:
acquiring a medical image of a subject;
by using a machine learning model, predicting a possibility of occurrence of a lesion of the subject based on the acquired medical image; and
outputting a result of the predicting,
wherein the machine learning model is a model trained with a plurality of training medical images and a risk of occurrence of the lesion associated with each of the plurality of training medical images.

18. The information processing system according to claim 17, wherein the plurality of training medical images include a high-risk group training medical image and a low-risk group training medical image, and
the high-risk group training medical image includes a first training medical image of a lesion region of a patient having the lesion, which was obtained before occurrence of the lesion in the lesion region.

19. The information processing system according to claim 17, wherein the plurality of training medical images include a high-risk group training medical image and a low-risk group training medical image, and
the high-risk group training medical image includes a second training medical image of a non-lesioned region of a patient having the lesion.

20. The information processing system according to claim 17, wherein the machine learning model includes:
a first classifier trained to classify the plurality of training medical images into a high-risk group training medical image or a low-risk group training medical image; and
a second classifier trained to classify the classified high-risk group training medical images into a plurality of classes.
